# EUROPÄISCHE PATENTSCHRIFT

(11) **EP 0 689 467 B1**
(45) Veröffentlichungstag und Bekanntmachung des Hinweises auf die Patenterteilung: **14.02.1996**
(21) Anmeldenummer: 93924039.6
(22) Anmeldetag: 13.10.1993
(51) Int. Cl.: A61M 25/00, A61M 5/00

(54) **KATHETER ZUR INJEKTION VON ARZNEIMITTELN**
CATHETER FOR INJECTING MEDICAMENTS
CATHETER UTILISE POUR INJECTER DES MEDICAMENTS

(30) Priorität: 21.10.1992 DE 4235506
(43) Veröffentlichungstag der Anmeldung: 03.01.1996
(73) Patentinhaber: Bavaria Medizin Technologie GmbH, D-82234 Wessling/Oberpfaffenhofen (DE)
(72) Erfinder: Gifford, Hanson, D-82211 Widdersberg (DE); Pösel, Andreas, D-81377 München (DE); Höfling, Berthold, D-82234 We ling (DE)
(74) Vertreter: Fleuchaus, Leo, Dipl.-Ing.
(86) Internationale Anmeldenummer: EP9302829
(87) Internationale Veröffentlichungsnummer: WO9408653

(56) Entgegenhaltungen:
- WO-A-92/10142
- DE-U- 8 913 761
- US-A- 3 598 119
- US-A- 4 578 061

## Beschreibung

Die Erfindung betrifft Katheter gemäß dem Oberbegriff des Anspruchs 1.

Zur medizinischen Behandlung von Patienten sind Systeme sinnvoll, mit denen Arzneimittel an der erkrankten Stelle im Körper direkt verabreicht werden können. Zur Behandlung innerhalb von Hohlorganen, Körperhöhlen oder Blutbahnen, insbesondere Arterien, wurden verschiedene Katheter vorgeschlagen, mit denen Arzneimittel in die Nähe der zu behandelnden Stelle des Gewebes gebracht werden können. Die einfachsten Katheter dieser Art sind Infusionskatheter, die in der Nähe der Katheterspitze ein oder mehrere Löcher haben oder sogar perforiert sind. Diese werden ausgeweitet, damit sie guten Kontakt mit der Wand des Gefäßes kommen und somit die Arzneimittel unmittelbar dem Gewebe zuführen. So kann man einen angeoplastischen Ballon ("poröser Ballon") herstellen, der eine Anzahl kleiner Löcher enthält und beim Zuführen der Arzneimittel unter Druck aufgeblasen wird, sich an die Wand der Arterie anlegt und mit dem dann die Arzneimittel unter Druck in das Gewebe eingeführt werden.

Nachteilig bei derartigen Konzepten ist, daß das Arzneimittel nur für eine kurze Zeit einwirkt, und die Menge des zugeführten Arzneimittels unsicher ist. Wenn einfache Infusionskatheter benutzt werden, kann das Arzneimittel auch sehr schnell weggeschwemmt werden. Dadurch kann es vorkommen, daß eine Infusion für die Dauer von mehreren Tagen nötig ist, um eine fiktive Arzneimittelkonzentration an der zu behandelnden Stelle zu erreichen. Das bedeutet einen langen Aufenthalt im Krankenhaus und ein großes Risiko für Infektionen oder andere Komplikationen. Als nachteilig wird auch empfunden, daß die Applikation des Arzneimittels nicht gleichzeitig mit einer Dilatation in einem Behandlungsschritt erfolgen kann.

Durch die US-A-4 578 061 ist ein Katheter nach dem Oberbegriff des Anspruchs 1 bekannt. Bei diesem Katheter wird die Injektionsnadel in der Katheterspitze mit einem flexiblen Draht verschoben, der in demselben Lumen läuft, durch welches das Medikament zugeführt wird. Die Injektionsnadel ist nur im vorderen Teil hohl und hat am rückwärtigen Ende einen zur Seite geführten Verbindungskanal, der in ausgestoßener Lage der Nadel mit einem weiteren in der Katheterspitze angebrachten Verbindungskanal fluchten muß, damit das Medikament appliziert werden kann. Diese fluchtende Ausrichtung ist nur schwer sicherzustellen.

In der WO92/10142 wird ein Katheter zur Prostatabehandlung beschrieben, bei dem mehrere rohrförmige Nadeln aus dem Katheterkopf herausschiebbar sind, um Glasfaserelement und Thermomeßsonden im Gewebe zu plazieren.

Aufgabe der Erfindung ist es, einen Katheter zu schaffen, der es ermöglicht, unter Vermeidung der oben dargestellten Nachteile, Arzneimittel einfach und sicher an Stellen im Körperinneren vorzugsweise Körperhöhlen, Hohlorganen oder Gefäßen zu applizieren, an denen das Arzneimittel benötigt wird.

Die Aufgabe wird durch Katheter gemäß den Ansprüchen 1,6,8 und 12 gelöst. Weiterbildungen der Erfindung sind durch die abhängigen Unteransprüchen gekennzeichnet.

Erfindungsgemäß weist ein Katheter Nadeln oder Kanülen auf. Diese Nadeln werden in einer eingezogenen Position innerhalb des Außenschaftes gehalten. In dieser Position kann der Katheter in ein Hohlorgan oder die Blutbahn, insbesondere eine Arterie eingeführt werden. Ferner ist der Katheter mit einer Vorrichtung versehen, mit der die Nadeln aus dem Katheter hinausgeschoben werden können. Diese Bewegung wird eingeleitet und ausgeführt, wenn sich der Katheter in der gewünschten Position befindet. Dabei legen sich die Nadeln an die Arterien- oder Gefäßwand an oder dringen in diese ein. Die Nadeln sind Hohlnadeln mit einem Nadelkanal, durch den das Arzneimittel in das Gewebe eingeführt werden kann. Die in die Gefäßwand eindringenden Nadeln verursachen eine Perforation die wegen der Feinheit der Nadeln so gering ist, daß keine blutenden Verletzungen entstehen. Außerdem bietet sich damit die Möglichkeit, ein Arzneimitteldepot mit definierter Arzneimittelmenge im Gewebe zu setzen.

Zum Verschieben der Nadeln kann ein im Katheter in Längsachse gegenüber dem Außenschaft verschiebbarer Innenschlauch vorgesehen sein, an dem die Nadeln so befestigt sind, daß der Innenschlauch diese an die Gefäßwand oder in das Gewebe schiebt. Eine andere Möglichkeit ist die Befestigung der Nadeln an einer ballonähnlichen Hülle, die sich unter Druck ausdehnt. Die Verschiebung der Nadeln wird bei dieser Ausführung durch aufgebrachten Druck in der Hülle bewirkt. Schließlich ist auch vorgesehen, die Nadeln mit Verlängerungen zu verwenden und diese insgesamt im Katheter zu verschieben.

Das Arzneimittel oder das Fluid wird von dem Katheterende aus zugeführt, das außerhalb des menschlichen Körpers liegt. Soll das Arzneimittel nicht zu tief in die Gefäßwand eindringen, ist es zweckmäßig, die Nadelspitzen so auszubilden, daß die angeschliffenen Stirnflächen, die an der Gefäßwand, z. B. einer Arterie, anliegen, nur leicht in diese eindringen oder möglichst parallel zur Wand zu liegen kommen. Das erreicht man dadurch, daß die Stirnflächen an den ausgefahrenen Nadelspitzen möglichst parallel zur Katheterachse verlaufen. Um eine bevorzugte Applikationsweise sicherzustellen, ist vorgesehen, daß die Nadeln auch etwas in das Gewebe eindringen. Daher sollte die Normale auf die Stirnfläche nicht genau parallel zur Nadelachse liegen, sondern zumindest um einen kleinen Winkel abweichen, damit die Nadeln nicht nur flach am Gewebe anliegen, sondern mit der Spitze geringfügig in das Gewebe eingedrückt werden können. Die bei der Handhabung angewandte Kraft und der Winkel bestimmen dann, wie tief die Nadelspitzen in das Gewebe eindringen, so daß eine gezielte Injektion in die Tiefe z. B. einer Gefäßwand lokal begrenzt möglich ist. Selbstverständlich ist die Nadelspitze der gewünschten Applikationsart entsprechend anzuschleifen und kann z.B. stumpf, abgerundet oder mit einer Facette versehen ausgebildet sein.

Gemäß einer Weiterbildung in der Erfindung werden Nadeln verwendet, die eine mechanische Vorspannung bzw. am vorderen Bereich eine Eigenkrümmung aufweisen, so daß die Nadeln, wenn sie mit einer Betätigungsvorrichtung nach außen verschoben werden, in Richtung auf die Wand austreten. Werden die Nadeln flexibler ausgeführt, so daß die Vorspannung nicht ausreicht, um die Nadeln vollständig in Richtung Wand zu führen, kann man auch an der Katheterspitze Nadelführungen vorsehen, mit denen die Nadeln umgebogen und gezielt zur Wand geschoben zu werden.

In einem Ausführungsbeispiel ist der Bewegungsmechanismus ein in Katheterlängsrichtung beweglicher Multilumenschlauch, der mehrere Kanäle hat, die sich von der Katheterspitze bis zum entgegengesetzten Ende des Katheters erstrecken. In diesen Kanälen sind an der Katheterspitze unter Vorspannung gebogene Nadeln befestigt. Durch Bewegung des Multilumenschlauchs in dem Katheteraußenschaft werden die Nadeln in Längsrichtung verschoben und bewegen sich aufgrund ihrer Vorspannung an die Gefäßwand. Zur Betätigung ist an dem der Katheterspitze gegenüberliegenden Ende eine Vorrichtung befestigt, die im wesentlichen aus zwei Teilen besteht. Dabei ist an dem ersten Teil der Katheteraußenschaft befestigt und an dem zweiten Teil der Multilumenschlauch.

Beide Teile werden mit einer Feder auseinandergehalten, so daß sich in Ruhepositition die Nadeln innerhalb des Außenschaftes des Katheters befinden. Beim Zusammendrücken beider Teile wird der Multilumenschlauch in dem Außenschaft nach vorne verschoben, so daß sich die Nadeln aus der Katheterspitze herausbewegen.

Eine ähnliche Vorrichtung kann auch dann zur Betätigung verwendet werden, wenn die Nadeln durch eine mit Druck beaufschlagte ballonartige Hülle nach außen gedrückt werden sollen, wobei das Zusammendrücken der beiden Teile der Betätigungsvorrichtung dann den entsprechenden Druck aufbaut.

Außerdem ist vorgesehen, daß die Nadeln mit rohrförmigen Verlängerungen in ihrer gesamten Länge durch den Katheter bis zur Betätigungsvorrichtung verlaufen und mit dieser im Katheter längsverschiebbar sind, um die Nadeln aus der Katheterspitze hinauszuschieben. Das Arzneimittel wird zur Applikation in der Betätigungsvorrichtung unter Druck in die Nadelverlängerungen eingeleitet.

Bei einer weiteren Ausführungsform der Erfindung ist unmittelbar vor dem Injektionsteil des Katheters ein herkömmlicher Ballon angebracht, wobei vorzugsweise der Führungsdraht außerhalb des Katheters vorgesehen ist und lediglich im Bereich des Ballons gemäß der US-PS 5,154,725 durch ein Innenlumen geführt wird (Monorail). Selbstverständlich ist es auch möglich den Führungsdraht durch den gesamten Katheterschaft, den Injektionskopf und den Ballon verschiebbar zu gestallten.

Damit läßt sich in vorteilhafter Weise die medikamentöse Gewebebehandlung mit der Dilatation in einem Behandlungsschritt verbinden, d.h. es ist kein Katheterwechsel nötig, was die Dauer des Eingriffs und die Patientenbelastung erheblich verringert. Im übrigen wird durch den kombinierten Injektions-Ballonkatheter dem Arzt die Möglichkeit geboten, die Entscheidung über die Art der Behandlung erst zu treffen, wenn der Katheter bereits eingeführt ist. Ein weiterer Vorteil wird auch darin gesehen, daß sich durch die Vorschaltung des Ballons vor den Injektionskopf eine sehr weiche Passagenerweiterung ergibt. Nach dem Injektionsvorgang kann bedarfsweise nochmals mit dem Ballon dilatiert werden, um die Verteilung des Arzneimittels zu optimieren und eventuelle mechanische Alterationen durch den Katheter zu glätten. Schließlich ist eine Behandlung mit nur einem Injektions-Ballonkatheter kostengünstiger als eine Behandlung mit zwei separaten Kathetern für denselben Zweck.

Weitere Vorteile und Merkmale der Erfindung ergeben sich auch aus der nachfolgenden Beschreibung von Ausführungsbeispielen in Verbindung mit den Patentansprüchen und der Zeichnung. Es zeigen:
- Fig. 1: eine schematische Darstellung eines erfindungsgemäßen Katheters mit Nadeln in einer eingezogenen Position;
- Fig. 2: den erfindungsgemäßen Katheter gemäß Fig. 1, mit nach außen verschobenen Nadeln;
- Fig. 3: eine Betätigungsvorrichtung zum Verschieben eines Multilumenschlauches im Katheter;
- Fig. 4: einen Radialschnitt durch eine andere Ausführungsform der Erfindung;
- Fig. 5: eine weitere Ausführungsform des erfindungsgemäßen Katheters;
- Fig. 6: eine Betätigungseinrichtung zum Verschieben der Nadeln beim Katheter gem. Fig. 5;
- Fig. 7: einen maßstäblich vergrößerten Schnitt längs der Linie VII-VII der Fig. 5.
- Fig. 8: einen Schnitt durch den Kopfteil eines erfindungsgemäß kombinierten Injektions-Ballonkatheters längs der Linie VIII - VIII der Fig. 11;
- Fig. 9: eine Betätigungsvorrichtung zum Längsverschieben der Injektionsnadeln im Katheter mit Anschlüssen für die Medikation und die Inflation/Deflation des Ballons;
- Fig. 10: einen Schnitt längs der Linie X-X der Fig. 8;
- Fig. 11: einen Schnitt längs der Linie XI-XI der Fig. 8.

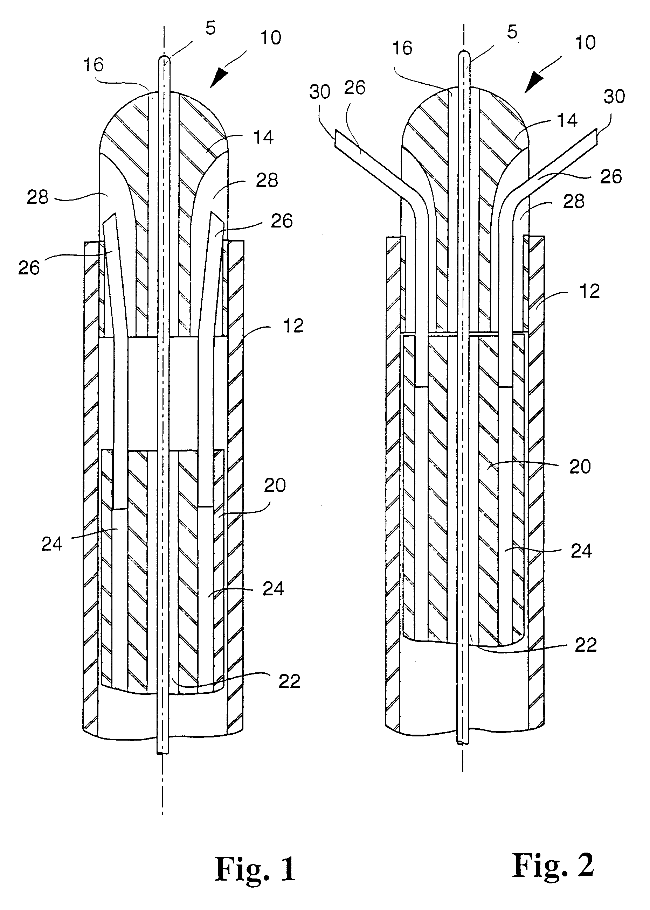

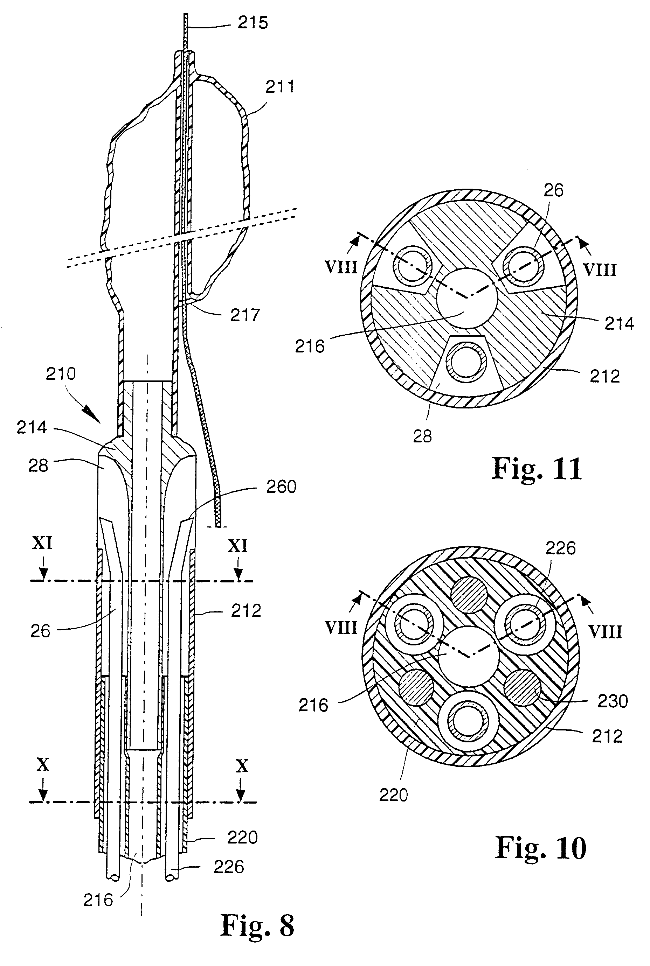

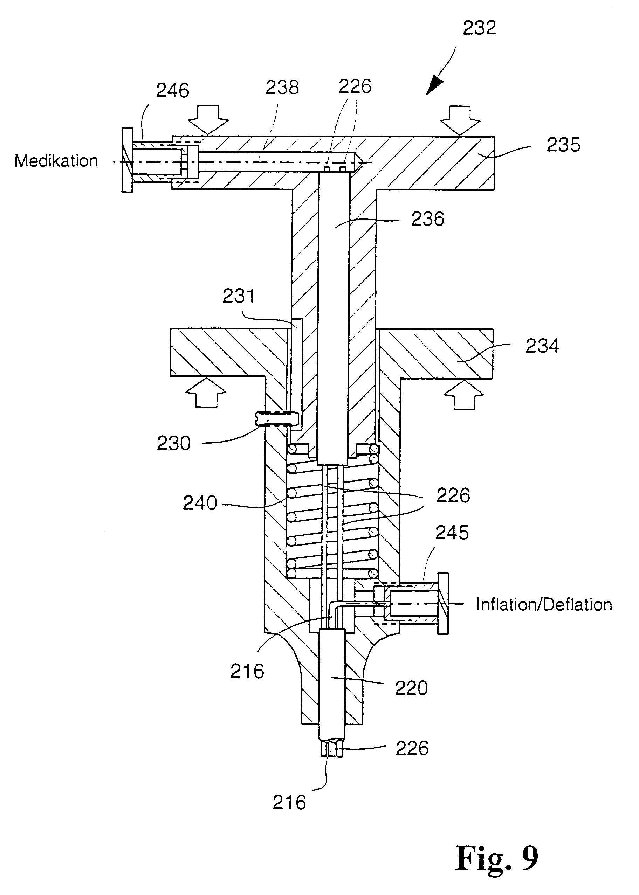

Fig. 1 zeigt einen Katheter 10, der auf einem Führungsdraht 5 in bekannter Weise in den Körper eingeführt werden kann. Der Katheter 10 weist einen Außenschaft 12 auf, an dessen Ende eine Katheterspitze 14 befestigt ist. Die Katheterspitze 14 kann durch Schweißen oder Kleben mit dem Außenschaft 12 fest verbunden sein. Die Katheterspitze 14 hat einen in Längsrichtung verlaufenden Kanal 16, durch den der Führungsdraht 5 hindurchgeführt ist. Weiter befindet sich innerhalb des Außenschaftes 12 ein Innenschlauch 20, der in Längsrichtung verschiebbar ist. Dieser Schlauch weist im Zentrum einen Kanal 22 auf, durch den der Führungsdraht 5 hindurchgeführt ist. Weiter sind in dem Innenschlauch Kanäle 24 (Lumina) vorgesehen, an deren katheterspitzenseitigem Ende Hohlnadeln 26 befestigt sind. Die Katheterspitze 14 ist mit in Längsrichtung verlaufenden Nuten oder Vertiefungen geeigneter Formgebung (siehe Fig. 11) versehen, die in Öffnungen 28 enden. Diese Öffnungen können sowohl seitlich als auch in einer abgerundeten oder konischen Katheterspitze austreten.

Die Hohlnadeln 26 stehen unter einer mechanischen Vorspannung, die sie radial an dem Außenschaft 12 drückt, dabei werden sie seitlich durch die Nutenwände von Öffnungen 28 gegen Verschwenken gehalten. Beim Vorschieben des inneren Schlauches 20 im Außenschaft 12 werden die Nadeln nach vorne geschoben und können sich aufgrund der Vorspannung oder auch durch ein Verbiegen beim Verschieben entlang der Innenwand der Öffnungen 28 nach außen bewegen, wo sie sich an die Gefäßwand anlegen oder in diese eindringen bzw. diese perforieren, um in die Adventitia zu gelangen, je nach dem wie es der Operateur aufgrund seiner Erfahrung vorsieht. Danach kann durch die Kanäle 24 und nicht gezeigten Kanälen in den Hohlnadeln 26 ein Arzneimittel dem Gewebe zugeführt werden. Das Arzneimittel tritt dann an der Stirnfläche 30 der Nadeln 26 aus. Die Stirnfläche 30 der ausgefahrenen Nadeln ist vorzugsweise so angeordnet, daß sie in Längsrichtung parallel zur Katheterachse liegt. Das bedeutet, Fläche 30 liegt etwa flach an der Gefäßwand an und kann nur unter größerem Kraftaufwand tiefer in das Gewebe eingeführt werden. Will man tiefer in das Gewebe eindringen, empfiehlt es sich, die Normale der Flächen 30 abweichend von der Senkrechten zur Katheterachse anzuordnen, so daß die Nadeln in das Gewebe eindringen. Selbstverständlich können auch andere dem Anwendungszweck angepaßte Nadelanschliffe für ein tieferes Eindringen in das Gewebe verwendet werden. Bei dem Ausführungsbeispiel gemäß Fig. 1 und Fig. 2 sind mehrere Nadeln 26 vorgesehen, die an mehreren Seiten des Katheters 10 aufgrund der Verschiebung des Innenschlauches 20 austreten, so daß der Katheter allseitig an der Gefäßwand abgestützt ist und zentrisch gehalten wird. Dadurch liegen die Flächen 30 definiert an der Wand an oder die Nadeln dringen bei entsprechendem Nadelanschliff in das Gewebe ein, und ein unbeabsichtigtes Wegschwemmen des Arzneimittels bzw. ein schlechter Kontakt einer Stirnfläche 30 an der Gefäßwand wird vermieden.

Zum guten Abstützen sollten mindestens 3 Nadeln verwendet werden. Es ist jedoch jede beliebige Anzahl von Nadeln möglich, die sowohl gleichmäßig als auch ungleichmäßig über den Querschnitt verteilt sein können. Bei mehr Nadeln kann auch der Durchfluß des Arzneimittels erhöht werden. Bei sehr dünnen Adern bzw. Gefäßen kann es jedoch sein, daß nicht genügend Platz für einen größeren Katheter vorhanden ist; dann wird man sich mit einer einzigen Nadel begnügen.

Sind mehrere Nadeln 26 wegen der besseren Stabilität bzw. des höheren Arzneimitteldurchflusses nötig, so ist der Innenschlauch 20 vorzugsweise ein Multilumenschlauch.

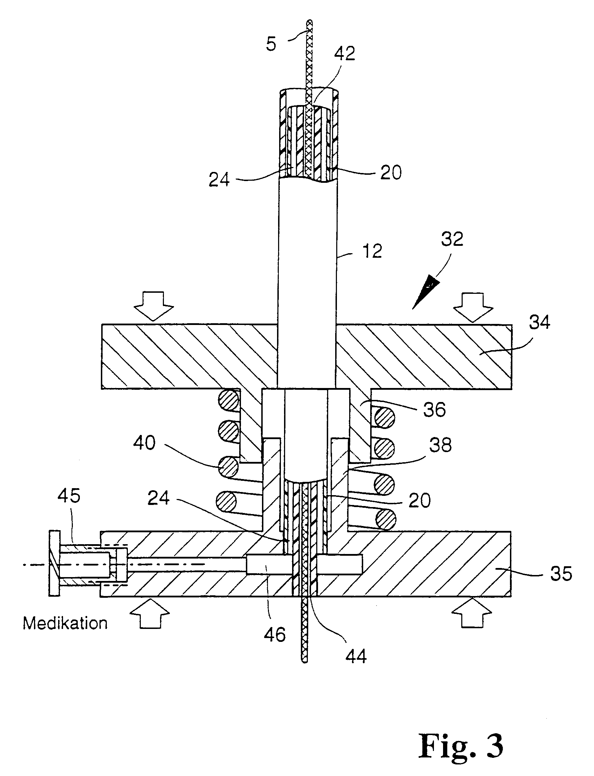

Fig. 3 zeigt die Betätigungsvorrichtung 30, wie sie verwendet werden kann, um die Verschiebung der Nadeln bei einem Katheter gemäß den Fig. 1 und Fig. 2 zu bewirken. Diese Betätigungsvorrichtung besteht im wesentlichen aus zwei Teilen 34 und 35. Das Teil 34 bleibt relativ zum Katheter fixiert und soll im folgenden als statisches Teil 34 bezeichnet werden. Gegenüber diesem ist ein bewegliches Teil 35 angeordnet. Im statischen Teil 34 ist der Außenschaft 12 befestigt und im beweglichen Teil 35 der Innenschlauch 20. Eine Führung 36 am statischen Teil 34 und eine Führung 38 am beweglichen Teil 35 greifen so ineinander, daß sich der Innenschlauch 20 nur axial zum Außenschaft 12 verschieben kann. Eine Feder 40 drückt die Teile 34 und 35 auseinander. Der Abstand der Stirnseite der Führung 38 vom gegenüberliegenden Anschlag ist so gewählt, daß sich die Nadeln 26 sicher in der eingezogenen Position gemäß Fig. 1 befinden. Wird das bewegliche Teil 35 und das statische Teil 34 zusammengedrückt, bewegt sich der Innenschlauch axial in Richtung Katheterspitze, so daß eine Position gemäß Fig. 2 erreicht wird, in der die Nadeln aufgrund ihrer Vorspannung an die Gefäßwand geführt werden.

Aus Fig. 3 ist zu erkennen, daß der Innenschlauch 20 im Teil 35 befestigt ist, wobei das Innenlumen 42 weitergeführt und den Führungsdraht 5 aus dem Innenlumen nach hinten durch eine Öffnung 44 heraustreten läßt. Das Arzneimittel wird durch einen seitlichen Anschluß 45 zugeführt und fließt durch einen Verteilerraum 46 über die Kanäle 24 bis zu den Nadeln 26.

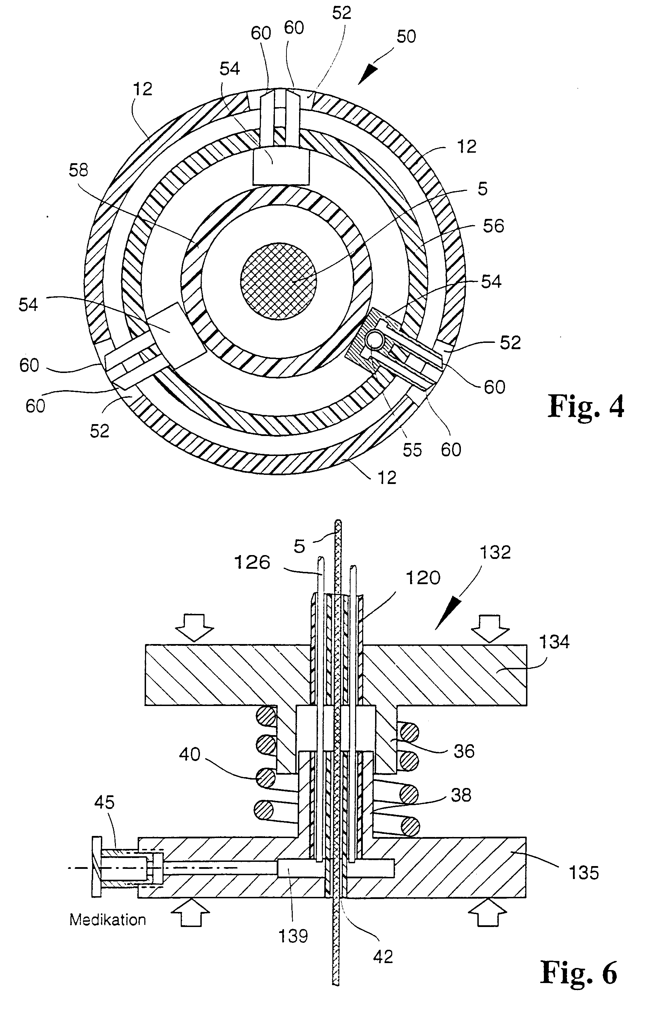

In Fig. 4 ist ein Querschnitt durch eine andere Ausführungsform des Katheters gezeigt. Dieser Katheter 50 hat einen Außenschaft 12. Im Zentrum des Katheters ist ein Führungsdraht 5 eingezeichnet. Der Außenschaft 12 hat Löcher 52, durch welche Nadeln 54 aus dem Innern des Katheters nach außen verschoben werden können. Die Nadeln 54 sind an einem inneren Schlauch 56 befestigt. Zwischen dem inneren Schlauch 56 und dem Führungsdraht 5 ist noch ein weiterer Schlauch 58 angeordnet. Der innere Schlauch 56 ist so elastisch, daß er sich bei einem Druckaufbau zwischen Schlauch 56 und Schlauch 58 ballonartig aufbläst und an die äußere Hülle 12 anlegt. Dadurch werden die Nadeln 54 durch die Löcher 52 hinausgestoßen und können sich an die Gefäßwand anlegen bzw. in diese eindringen. In diesem Beispiel ist eine Form der Nadeln gezeigt, bei denen die Stirnfläche 60 anders ausgebildet ist als bei den Nadeln gemäß Fig. 1 und Fig. 2. Bei diesen Nadelflächen 60 steht die Flächennormale nicht senkrecht auf der Katheterachse, wodurch es für die Nadelspitzen möglich ist, tiefer in die Gefäßwand oder das Gewebe einzudringen. Die Vorteile wurden vorstehend schon beschrieben. Es ist aber auch möglich, die Nadeln 56 mit Stirnflächen 60 auszustatten, die wie die Stirnflächen 30 bei der ersten Ausführungsform flach an der Gefäßwand anliegen.

In dem in Fig. 4 gezeigten Beispiel werden die Nadeln durch getrennte an die Bohrung 55 in der Nadel 54 angeschlossene Kanäle mit Arzneimittel versorgt und der Hohlraum zwischen den Schläuchen 56 und 58 unabhängig davon unter Druck gesetzt. Damit wird ein wesentlicher Nachteil von ballonartig aufblasbaren Kathetern nach dem Stand der Technik vermieden, indem bei einem hohen Inflationsdruck auch ein niedrigerer Applikationsdruck für das Arzneimittel verwendet werden kann. Außerdem kann durch das individuell wählbare Verschieben der Nadeln bzw. durch die unterschiedliche Eindringtiefe das Arzneimittel unterschiedlich plaziert werde, nämlich in den Innenschichten des Gefäßes, in die Gefäßwand oder auch als Depot in die Gefäßumgebung. Im übrigen bietet sich der Vorteil, daß sich die Dilatation und die Arzneimittelinjektion als zwei klar separierte Arbeitsvorgänge individuell kontrollieren lassen. Für diesen Fall ist eine Betätigungsvorrichtung bekannter Art anwendbar, über welche das Druckmedium und das Arzneimittel getrennt zugeführt werden können.

Bei einer solchen Vorrichtung zum Betrieb des Katheters 50 wird der Inflationsdruck zum Aufweiten des inneren Schlauches 56 über einen mit dem Zwischenraum zwischen den Schläuchen 56 und 58 verbundenen Anschluß zugeführt, womit die Dilatation ausgeführt wird. Das Arzneimittel wird über einen separaten Anschluß an der Betätigungsvorrichtung den im Zwischenraum zwischen den Nadeln verlaufenden und mit den Nadeln 54 verbundenen Kanälen zugeführt. Beide Ausführungsbeispiele der Erfindung haben den Vorteil, daß durch die Verwendung der Nadeln 26 bzw. 54 eine gezielte Injektion in eine Gefäßwand und/oder deren Umgebung möglich ist. Durch eine entsprechende Ausbildung der Stirnflächen 30 und 60 läßt sich zudem auch die Eindringtiefe der Nadel und damit des Arzneimittels beeinflussen.

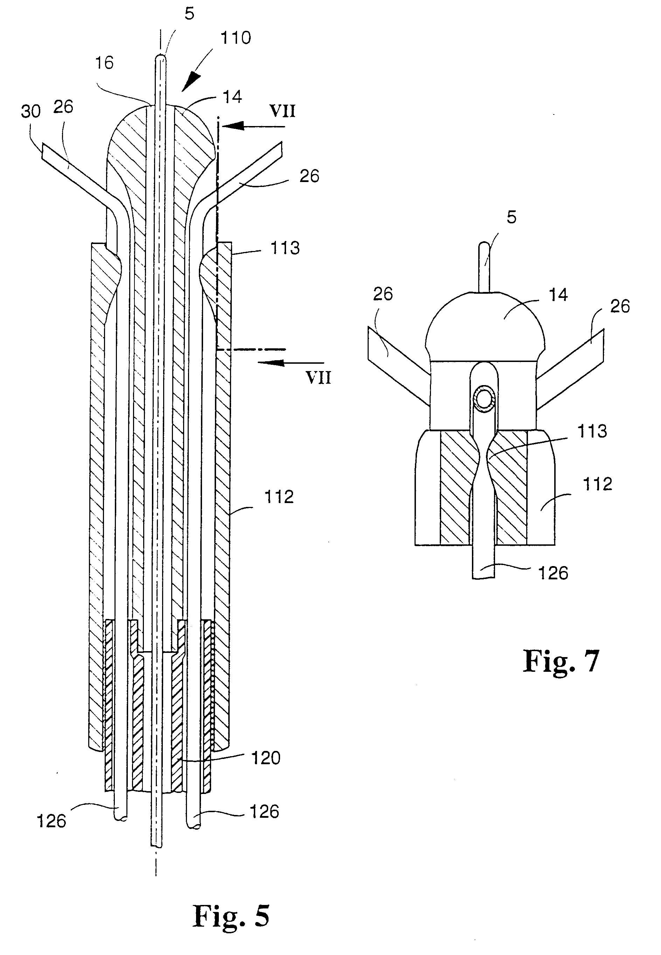

In Fig. 5 ist eine weitere Ausführungsform des Katheters 110 gezeigt, bei der die elastisch nach außen vorgespannten Nadeln 26 in rohrförmige Verlängerungen 126 übergehen. Diese Verlängerungen können einstückig mit den Nadeln ausgebildet sein und verlaufen durch die Kanäle eines als Multilumenschlauch ausgeführten Katheters, wobei der Durchmesser der Kanäle groß genug ist, um die Verlängerungen 126 der Nadeln 26 leicht in ihnen gleiten zu lassen.

Am vorderen Ende des Multilumenschlauches ist ein Außenschaft 112 befestigt, in dem die Nadeln geführt und in welchen die Nadeln zurückgezogen werden können. Die mit dem Außenschaft 112 zusammenwirkende Katheterspitze 14 ist, wie bereits erläutert, so ausgebildet, daß sie die elastisch vorgespannten Nadeln 26 in der gewünschten Weise beim Ausstoßen führt. Dabei ist auch gemäß den Fig. 5 und 7 vorgesehen, daß der Außenschaft 112 für jede Nadel am vorderen Innenbereich mit einer Verdickung 113 versehen ist, die zusätzlich zu den nutenförmigen Ausnehmungen in der Katheterspitze die Nadel führt und gegen ein Verschwenken beim Austreten sichert. Durch die Katheterspitze verläuft der Führungsdraht 5 in bekannter Weise. Vorzugsweise sind die Katheterspitze und der Außenschaft aus Metall oder einem harten, gut gleitenden Kunststoffmaterial hergestellt.

Zur Verschiebung der Nadeln 26 mit ihren Verlängerungen 126 dient Betätigungsvorrichtung 132 gemäß Fig. 6. Sie ist im wesentlichen wie die Betätigungsvorrichtung 30 gemäß Fig. 3 aufgebaut. Am feststehenden Teil 134 ist das hintere Ende des Multilumenschlauches 120 befestigt, während die Nadelverlängerungen 126 bis zum beweglichen Teil 135 verlaufen und dort derart gehaltert sind, daß durch die freien Lumina das Fluid oder Arzneimittel von dem Verteilervolumen 139 aus unter Druck zugeführt werden kann. Die Handhabung der Betätigungsvorrichtung entspricht der gemäß Fig. 3, wobei die Vorrichtung griffig ausgebildet ist, um sie mit einer Hand bedienen zu können.

Es ist vorgesehen, eine geradzahlige als auch eine ungeradzahlige Anzahl von Nadeln zu verwenden, wobei diese sowohl gleichmäßig als auch ungleichmäßig über den Katheterquerschnitt verteilt sein können.

Bei der Verwendung von z.B. zwei Nadeln kann auch vorgesehen sein, daß der Führungsdraht außermittig geführt ist, beispielsweise durch einen dritten Kanal des Multilumenschlauches. dann kann der Führungsdraht auch außermittig aus der Katheterspitze austreten.

Ein Katheter der vorbeschriebenen Art hat vorzugsweise einen Durchmesser zwischen etwa 0,5 mm und 5 mm, bei einer Länge der Katheterspitze von etwa 1 mm bis 10 mm.

In Fig. 8 ist eine weitere Ausführungsform der Erfindung gezeigt, bei der vor dem Injektionsteil 210 ein Ballon 211 angeordnet ist. Dabei verläuft der Führungsdraht 215, wie bei den sog. Monorail-Kathetern üblich, nur durch das Innere des Ballonbereiches. Die Katheterspitze 214 für den Injektionsteil ist im wesentlichen, wie bei der Ausführungsform gemäß Fig. 5, aufgebaut, wobei die Katheterspitze 214 mit dem Multilumenschlauch 220 über einen Außenschaft 212 verbunden ist. Die Hohlnadeln 26 verlaufen von der Katheterspitze 214 durch den Multilumenschlauch 220 bis zur Betätigungseinrichtung gemäß Fig. 9. Diese Hohlnadeln 26 sind - obwohl nicht dargestellt - mit dem nachfolgend erläuterten Schubmechanismus verbunden.

Das Medium zum Inflatieren wird durch das Innenlumen 216 des Multilumenschlauches 220 zugeführt, das als Bohrung durch die Katheterspitze 214 weitergeführt im Lumen zum Inflatieren des Ballons 211 endet.

In Fig. 10 und 11 sind zwei Schnitte durch die Katheterspitze dargestellt, wobei Fig. 10 einen vom Außenschaft 212 umgebenen Multilumenschlauch 220 zeigt, in welchem die Kanäle für die Hohlnadeln und deren Verlängerungen 226 bei der dargestellten Ausführungsform in einer Dreieckzuordnung zueinander angeordnet sind. Zwischen den Kanälen für die Hohlnadelverlängerungen sind in das Material des Multilumenschlauches Versteifungsdrähte 230 eingespritzt. Durch diese Versteifungsdrähte wird sichergestellt, daß sich der Katheter beim Verschieben der Hohlnadel innerhalb des Katheters nicht unkontrolliert seitlich ausbeult.

In Fig. 9 ist die Betätigungsvorrichtung bzw. der Schubmechanismus 232 dargestellt, mit dem die Hohlnadeln bzw. deren Verlängerungen 226 durch den Multilumenschlauch 220 zur Injektion verschiebbar sind. Zu diesem Zweck besteht der Schubmechanismus aus einem statischen Teil 234 und einem beweglichen Teil 235, der mit Hilfe einer Feder 240 in einer Position gehalten wird, in der die Hohlnadeln in die Katheterspitze 214 zurückgezogen sind. Die Hublänge, d.h. die Weglänge um welche die Hohlnadeln aus der Katheterspitze herausverschoben werden können, wird durch einen Anschlagstift 230 festgelegt, der im statischen Teil 234 befestigt ist und in eine Nut 231 im beweglichen Teil 235 eingreift. Durch die Länge dieser Nut 231 wird der Nadelhub festgelegt.

In das vordere Ende des statischen Teils 234 ist der Multilumenschlauch 220 eingeklebt und unverrückbar gehalten. Das gleiche trifft für den im beweglichen Teil 235 verlaufenden Abschnitt 236 eines Multilumenschlauches zu, in den außerdem die in einen Verteilerraum 238 mündenden Enden der Verlängerungen 226 ebenfalls eingeklebt sind. Damit ist bei einer Betätigung des Schubmechanismus im Sinne der angegebenen Pfeile eine Verschiebung der Hohlnadeln aus der Katheterspitze heraus möglich.

Das Innerlumen 216 wird im statischen Teil 234 seitlich herausgeführt und endet in einem Anschluß 245, durch welchen das Medium für die Inflation in herkömmlicher Weise zugeführt wird. Durch denselben Anschluß erfolgt auch die Deflation.

Ein entsprechender Anschluß 246 ist am beweglichen Teil 235 angeordnet und mündet in den Verteilerraum 238. Durch diesen Anschluß 246 erfolgt die Medikation in herkömmlicher Weise.

Im Interesse eines besseren Verständnisses und der besseren Erkennbarkeit sind die in den einzelnen Figuren dargestellten Teile in einem unterschiedlichen Maßstab gezeichnet.

## Patentansprüche

1. Katheter (10) zur Injektion eines Fluids bzw. eines Arzneimittels in Hohlorgane oder Körperhöhlen, insbesondere in Koronargefäße und Arterien mit einer in Arterien einführbaren Katheterspitze (14) und einem Katheteraußenschaft (12), einer in der Katheterspitze (14) verschiebbar angeordneten Injektionsnadel (26), wobei die Nadelspitze in einer eingezogenen Position innerhalb der Katheterspitze liegt und für die Applikation des Fluids oder des Arzneimittels in einer herausgeschobenen Position über die Katheterspitze vorsteht, einer am extrakorporalen Ende angebrachten Betätigungsvorrichtung mit einem ersten und zweiten Teil, wobei der Katheteraußenschaft and dem einen Teil der Betätigungsvorrichtung befestigt ist, und wobei die beiden Teile durch Gegenkraft auseinander gehalten werden, mit welcher Betätigungsvorrichtung die Nadelspitze durch den Katheteraußenschaft (12) verschiebbar ist, und mit einer in der Katheterspitze (14) vorgesehenen Öffnung (28) mit einer Nadelführung, die sowohl einen nach seitlich als auch nach vorn gerichteten Austritt der Injektionsnadel (26) zuläßt,
**dadurch gekennzeichnet**,
daß mehrere Injektionsnadeln vorgesehen sind, die an einem im Katheter (10) in Längsachse verschiebbaren Schlauch (20) befestigt sind, der als ein in dem Katheteraußenschaft (12) verschiebbarer Multilumenschlauch (20) ausgebildet ist,
daß der Multilumenschlauch (20) an dem zweiten Teil (35) der Betätigungsvorrichtung (32) befestigt ist,
daß beim Zusammendrücken der beiden Teile gegen die Gegenkraft die im Multilumenschlauch (20) befestigte Injektionsnadeln (26) von der zurückgezogenen in die nach außen vorstehende Position bewegt werden, wobei das Fluid oder das Arzneimittel über den anderen Teil (35) und den Multilumenschlauch (20) gleichzeitig zuführbar ist,
und daß ein Führungsdraht (5) sowohl innerhalb des Multilumenschlauches als auch durch die Katheterspitze (14) geführt ist.

2. Katheter nach Anspruch 1,
**dadurch gekennzeichnet**,
daß drei in einem Winkelabstand von 120° angeordnete Injektionsnadeln Verwendung finden.

3. Katheter nach Anspruch 1 oder 2,
**dadurch gekennzeichnet**,
daß die Nadelspitzen eine Stirnfläche (30) haben, die in der herausgeschobenen Position etwa parallel zur Katheterachse verläuft.

4. Katheter nach einem der Ansprüche 1 bis 3,
**dadurch gekennzeichnet**,
daß die Nadelspitzen eine Stirnfläche haben, deren Normale in der herausgeschobenen Position unter einem Winkel zur Radialrichtung des Katheters verläuft.

5. Katheter nach einem oder mehreren der Ansprüche 1 bis 4,
**dadurch gekennzeichnet**,
daß die in Längsrichtung verschiebbaren Nadeln (26) unter einer Vorspannung oder Eigenkrümmung stehen,
und daß sich die Nadelspitzen beim Verschieben der Injektionsnadeln (26) in Längsrichtung durch den Katheteraußenschaft (12) in eine seitlich vorstehende Position bewegen.

6. Katheter (10) zur Injektion eines Fluids bzw. eines Arzneimittels in Hohlorgane oder Körperhöhlen, insbesondere in Koronargefäße und Arterien mit einer in Arterien einführbaren Katheterspitze (14) und einem Katheteraußenschaft (12), einer in der Katheterspitze (14) verschiebbar angeordneten Injektionsnadel (26), wobei die Nadelspitze in einer eingezogenen Position innerhalb der Katheterspitze liegt und für die Applikation des Fluids oder des Arzneimittels in einer herausgeschobenen Position über die Katheterspitze vorsteht, einer am extrakorporalen Ende angebrachten Betätigungsvorrichtung mit einem ersten und zweiten Teil, wobei der Katheteraußenschaft an dem einen Teil der Betätigungsvorrichtung befestigt ist, und wobei die beiden Teile durch Gegenkraft auseinander gehalten werden, mit welcher Betätigungsvorrichtung die Nadelspitze durch den Katheteraußenschaft (12) verschiebbar ist, und mit einer in der Katheterspitze (14) vorgesehenen Öffnung (28) mit einer Nadelführung, die sowohl einen nach seitlich als auch nach vorn gerichteten Austritt der Injektionsnadel (26) zuläßt,
**dadurch gekennzeichnet**,
daß der Katheteraußenschaft als Multilumenschlauch (120) ausgebildet und an dem einen Teil (134) der Betätigungsvorrichtung (132) befestigt ist,
daß mehrere Injektionsnadeln (26) vorgesehen und mit rohrförmigen Verlängerungen (126) versehen sind, die durch jeweils ein Lumen des Multilumenschlauches (120) verlaufen und an dem anderen Teil (135) der Betätigungsvorrichtung (132) befestigt sind,
daß die im Multilumenschlauch (120) geführten Verlängerungen (126) beim Zusammendrücken der beiden Teile gegen die Gegenkraft die Injektionsnadeln (26) von der zurückgezogenen in die nach außen vorstehende Position bewegen, wobei das Fluid oder das Arzneimittel über den anderen Teil (135) sowie die Verlängerungen (126) den Injektionsnadeln (26) gleichzeitig zuführbar ist,
und daß ein Führungsdraht (5) sowohl innerhalb des Multilumenschlauches als auch durch die Katheterspitze geführt ist.

7. Katheter nach Anspruch 6,
**dadurch gekennzeichnet**,
daß die verschiebbaren Verlängerungen (126) der Injektionsnadeln (26) bis zur Katheterspitze verlaufen.

8. Katheter (10) zur Injektion eines Fluids bzw. eines Arzneimittels in Hohlorgane oder Körperhöhlen, insbesondere in Koronargefäße und Arterien mit einer in Arterien einführbaren Katheterspitze (14) und einem Katheteraußenschaft (12), einer in der Katheterspitze (14) verschiebbar angeordneten Injektionsnadel (26), wobei die Nadelspitze in einer eingezogenen Position innerhalb der Katheterspitze liegt und für die Applikation des Fluids oder des Arzneimittels in einer herausgeschobenen Position über die Katheterspitze vorsteht, einer am extrakorporalen Ende angebrachten Betätigungsvorrichtung mit einem ersten und zweiten Teil, wobei der Katheteraußenschaft an dem einen Teil der Betätigungsvorrichtung befestigt ist, und wobei die beiden Teile durch Gegenkraft auseinander gehalten werden, mit welcher Betätigungsvorrichtung die Nadelspitze durch den Katheteraußenschaft (12) verschiebbar ist und mit einer in der Katheterspitze (14) vorgesehenen Öffnung (28) mit einer Nadelführung, die sowohl einen nach seitlich als auch nach vorn gerichteten Austritt der Injektionsnadel (26) zuläßt,
**dadurch gekennzeichnet**,
daß der Katheteraußenschaft als Multilumenschlauch (220) ausgebildet und an dem einen Teil (234) der Betätigungsvorrichtung (232) befestigt ist,
daß mehrere Injektionsnadeln (260) vorgesehen und mit rohrförmigen Verlängerungen (226) versehen sind, die durch jeweils ein Lumen des Multilumenschlauches (220) verlaufend an dem anderen Teil (235) der Betätigungsvorrichtung (232) befestigt sind,
daß die im Multilumenschlauch (220) geführten Verlängerungen (226) beim Zusammendrücken der beiden Teile gegen die Gegenkraft die Injektionsnadeln (260) von der zurückgezogenen in die nach außen vorstehende Position bewegen, wobei das Fluid oder das Arzneimittel über den anderen Teil (235) und die Verlängerungen (226) den Injektionsnadeln (260) gleichzeitig zuführbar ist,
daß auf die der Injektion dienenden Katheterspitze (214) ein Ballon (211) aufgesetzt ist, der über einen Kanal (216) des Multilumenschlauches (220) inflatierbar und deflatierbar ist,
und daß ein Führungsdraht (215) im Ballon (211) durch ein Innenlumen (217) zur Spitze des Ballons geführt ist.

9. Katheter nach Anspruch 8,
**dadurch gekennzeichnet**,
daß der Führungsdraht (215) entweder innerhalb des Multilumenschlauches (220) und durch die Katheterspitze (214) sowie den Ballon (211), oder außerhalb des Multilumenschlauches (220) bis zum Ballon (211) und erst im Ballon durch das Innenlumen (217) zur Spitze geführt ist.

10. Katheter nach Anspruch 8,
**dadurch gekennzeichnet**,
daß in das Material des Multilumenschlauches Versteifungsdrähte (230) eingespritzt sind.

11. Katheter nach einem oder mehreren der Ansprüche 1 bis 10,
**dadurch gekennzeichnet**,
daß in der Katheterspitze (14; 214) Öffnungen (28) mit Nadelführungen angeordnet sind, welche die Injektionsnadeln beim Austritt sowohl nach seitlich als auch nach vorn gerichtet führen.

12. Katheter (10) zur Injektion eines Fluids bzw. eines Arzneimittels in Hohlorgane oder Körperhöhlen, insbesondere in Koronargefäße und Arterien mit einer in Arterien einführbaren Katheterspitze (14) und einem Katheteraußenschaft (12), einer in der Katheterspitze (14) verschiebbar angeordneten Injektionsnadel (54), wobei die Nadelspitze in einer eingezogenen Position innerhalb der Katheterspitze liegt und für die Applikation des Fluids oder des Arzneimittels in einer herausgeschobenen Position über die Katheterspitze vorsteht, einer am extrakorporalen Ende angebrachten Betätigungsvorrichtung mit einem ersten und zweiten Teil, wobei der Katheteraußenschaft an dem einen Teil der Betätigungsvorrichtung befestigt ist, und wobei die beiden Teile durch Gegenkraft auseinander gehalten werden, mit welcher Betätigungsvorrichtung die Nadelspitze durch die Katheterspitze verschiebbar ist, und mit einer in der Katheterspitze vorgesehenen Öffnung (52) mit einer Nadelführung, die sowohl einen nach seitlich als auch nach vorn gerichteten Austritt der Injektionsnadel (54) zuläßt,
**dadurch gekennzeichnet**,
daß mehrere Injektionsnadeln (54) vorgesehen und an einem inneren Schlauch (56) angebracht sind, der außerhalb von einem Innenschlauch (58) liegt, wobei der Zwischenraum mit Druck beaufschlagbar ist, um den inneren Schlauch (56) aufzuweiten und die Nadeln durch die Katheterspitze zu verschieben,
und daß ein Führungsdraht (5) sowohl innerhalb des Innenschlauches (56) als auch durch die Katheterspitze geführt ist.

## Claims

1. A catheter (10) for injecting a fluid or a pharmaceutical preparation into hollow organs or body cavities, more particularly coronary vessels and arteries, comprising a catheter tip (14) insertable into arteries and a catheter outer shaft (12), an injection needle (26) arranged so as to be displaceable in the catheter tip (14), the needle point, when in a retracted position, resting inside the catheter tip and, when in an extended position, protruding beyond the catheter tip for administering the fluid or the pharmaceutical preparation, further comprising an actuating device arranged at the extracorporeal end, having a first and a second part, the catheter outer shaft being secured to one part of the actuating device and the two parts being kept apart by a repulsive force, by means of which actuating device the needle point is displaceable through the catheter outer shaft (12), and comprising an aperture (28) provided in the catheter tip (14), said aperture having a needle guide which permits the injection needle (26) to protrude to the side as well as in the forward direction, characterised in that several injection needles are provided which are secured to a tube (20) displaceable in the catheter (10) along the longitudinal axis thereof, which tube is constructed as a multi-lumina tube (20) displaceable in the catheter outer shaft (12), in that the multi-lumina tube (20) is secured to the second part (35) of the actuating device (32), in that, when the two parts are pressed together against the repulsive force, the injection needles (26) fixed in the multi-lumina tube (20) are moved from the retracted into the outwardly protruding position; whereby the fluid or the pharmaceutical preparation may be supplied via the other part (35) and the multi-lumina tube (20) simultaneously, and in that a guide wire (5) is guided both inside the multi-lumina tube and through the catheter tip (14).

2. A catheter according to claim 1, characterised in that three injection needles are used which are arranged at an angular distance of 120°.

3. A catheter according to claim 1 or 2, characterised in that the needle points have an end face (30) which, when in the extended position, extends substantially parallel to the catheter axis.

4. A catheter according to one of claims 1 to 3, characterised in that the needle points have an end face whose normal line, when the points are in the extended position, extends at an angle to the radial direction of the catheter.

5. A catheter according to one or more of claims 1 to 4, characterised in that the needles (26) which are displaceable in the longitudinal direction are pretensioned or have a natural curvature, and in that the needle points, when the injection needles (26) are displaced, move in the longitudinal direction through the catheter outer shaft (12) into a position so as to be protruding to the side.

6. A catheter (10) for injecting a fluid or a pharmaceutical preparation into hollow organs or body cavities, more particularly coronary vessels and arteries, comprising a catheter tip (14) insertable into arteries and a catheter outer shaft (12), an injection needle (26) arranged so as to be displaceable in the catheter tip (14), the needle point, when in a retracted position, resting inside the catheter tip and, when in an extended position, protruding beyond the catheter tip for administering the fluid or the pharmaceutical preparation, comprising an actuating device arranged at the extracorporeal end, having a first and a second part, the catheter outer shaft being secured to one part of the actuating device and the two parts being kept apart by a repulsive force, by means of which actuating device the needle point is displaceable through the catheter outer shaft (12), and comprising an aperture (28) provided in the catheter tip (14), said aperture having a needle guide which permits the injection needle (26) to protrude to the side as well as in the forward direction, characterised in that the catheter outer shaft is in the form of a multi-lumina tube (120) and is secured to one part (134) of the actuating device (132), that several injection needles (26) are provided and have tubular extensions (126) which each extend through a lumen of the multi-lumina tube (120) and are secured to the other part (135) of the actuating device (132), that the extensions (126) guided in the multi-lumina tube (120) move the injection needles (26) from the retracted in the outwardly protruding position when the two parts are pressed together against the repulsive force, whereby the fluid or the pharmaceutical preparation may be supplied to the injection needles (26) via the other part (135) and the extensions (126) simultaneously, and that a guide wire (5) is guided both inside the multi-lumina tube and through the catheter tip.

7. A catheter according to claim 6, characterised in that the displaceable extensions (126) of the injection needles (26) extend right up to the catheter tip.

8. A catheter (10) for injecting a fluid or a pharmaceutical preparation into hollow organs or body cavities, more particularly coronary vessels and arteries, comprising a catheter tip (14) insertable into arteries and a catheter outer shaft (12), an injection needle (26) arranged so as to be displaceable in the catheter tip (14), the needle points, when in a retracted position, resting inside the catheter tip and, when in an extended position, protruding beyond the catheter tip for administering of the fluid or the pharmaceutical preparation, comprising an actuating device arranged at the extracorporeal end, having a first and a second part, the catheter outer shaft being secured to one part of the actuating device and the two parts being kept apart by a repulsive force, by means of which actuating device the needle point being displaceable through the catheter outer shaft (12), and comprising an aperture (28) provided in the catheter tip (14), said aperture having a needle guide which permits the injection needle (26) to protrude to the side as well as in the forward direction, characterised in that the catheter outer shaft is in the form of a multi-lumina tube (220) and is secured to one part (234) of the actuating device (232), that several injection needles (260) are provided and have tubular extensions (226) which are secured to the other part (235) of the actuating device (232) so that each extends through a lumen of the multi-lumina tube (220), that the extensions (226) guided in the multi-lumina tube (220) move the injection needles (260) from the retracted into the outwardly projecting position, when the two parts are pressed together against the repulsive force, whereby the fluid or the pharmaceutical preparation may be supplied to the injection needles (260) via the other part (235) and the extensions (226) simultaneously, that a balloon (211) is placed on to the catheter tip (214) used for the injection, which balloon may be inflated and deflated via a channel (216) of the multi-lumina tube (220), and that a guide wire (215) is guided through an internal lumen (217) in the balloon (211) up to the top of the balloon.

9. A catheter according to claim 8, characterised in that the guide wire (215) is guided up to the top either inside the multi-lumina tube (220) and then through the catheter tip (214) and the balloon (211), or is guided outside the multi-lumina tube (220) up to the balloon (211) and only then passes through the internal lumen (217) in the balloon.

10. A catheter according to claim 8, characterised in that reinforcing wires (230) are moulded into the material of the multi-lumina tube.

11. A catheter according to one or several of claims 1 to 10, characterised in that apertures (28) with needle guides are arranged in the catheter tip (14; 214) which guide the injection needles, when these protrude, both to the side and in the forward direction.

12. A catheter (10) for injecting a fluid or a pharmaceutical preparation into hollow organs or body cavities, more particularly coronary vessels and arteries, comprising a catheter tip (14) insertable into arteries and a ocatheter outershaft (12), an injection needle (54) arranged so as to be displaceable in the catheter tip (14), the needle point, when in a retracted position, resting inside the catheter tip and, when in an extended position, protruding beyond the catheter tip for administering the fluid or the pharmaceutical preparation, comprising an actuating device arranged at the extracorporeal end, having a first and a second part, the catheter outer shaft being secured to one part of the actuating device and the two parts being kept apart by a repulsive force, by means of which actuating device the needle point is displaceable through the catheter tip, and comprising an aperture (52) provided in the catheter tip, said aperture having a needle guide which permits the injection needle (54) to issue to the side as well as in the forward direction, characterised in that a plurality of injection needles (54) are provided and are arranged on an inner tube (56) which is positioned outside an internal tube (58), the space between the tubes being acted upon by pressure in order to widen the inner tube (56) and to push the needles through the catheter tip, and that a guide wire (5) is guided inside the inner tube (56) and through the catheter tip.

## Revendications

1. Cathéter (10) pour injecter, respectivement, un fluide ou un médicament dans des organes creux ou des cavités corporelles, en particulier dans des vaisseaux coronariens et des artères, comprenant une pointe (14) pouvant être introduite dans des artères et un fût extérieur (12) ; une aiguille d'injection (26) logée à coulissement dans la pointe (14) du cathéter, la pointe de l'aiguille étant située à l'intérieur de la pointe du cathéter dans une position rétractée, et faisant saillie au-delà de la pointe du cathéter dans une position déployée en vue de l'administration du fluide ou du médicament ; un dispositif d'actionnement implanté à l'extrémité extracorporelle et comportant des première et seconde parties, le fût extérieur du cathéter étant fixé à l'une des parties du dispositif d'actionnement, et les deux parties étant maintenues dissociées par une force antagoniste, dispositif d'actionnement à l'aide duquel la pointe de l'aiguille peut être animée d'un coulissement à travers le fût extérieur (12) du cathéter ; et un orifice (28) prévu dans la pointe (14) du cathéter et associé à un guide-aiguille qui autorise une sortie de l'aiguille d'injection (26) dirigée tant latéralement, que vers l'avant,
caractérisé par le fait
que plusieurs aiguilles d'injection sont prévues et sont fixées à un flexible (20) qui peut coulisser dans le cathéter (10), selon l'axe longitudinal, et est réalisé sous la forme d'un flexible (20) à plusieurs canaux intérieurs, pouvant coulisser dans le fût extérieur (12) du cathéter ;
que le flexible (20) à plusieurs canaux intérieurs est fixé à la seconde partie (35) du dispositif d'actionnement (32) ;
que, lors de la compression des deux parties s'opposant à la force antagoniste, les aiguilles d'injection (26) fixées dans le flexible (20) à plusieurs canaux intérieurs sont déplacées de la position rétractée à la position saillant vers l'extérieur, le fluide ou le médicament pouvant être simultanément délivré par l'intermédiaire de l'autre partie (35) et du flexible (20) à plusieurs canaux intérieurs ;
et qu'un fil métallique de guidage (5) est guidé tant à l'intérieur du flexible à plusieurs canaux intérieurs, qu'à travers la pointe (14) du cathéter.

2. Cathéter selon la revendication 1,
caractérisé par le fait
qu'on utilise trois aiguilles d'injection, agencées selon un espacement angulaire de 120°.

3. Cathéter selon la revendication 1 ou 2,
caractérisé par le fait
que les pointes des aiguilles présentent une face extrême (30) qui, dans la position déployée, s'étend à peu prés parallèlement à l'axe dudit cathéter.

4. Cathéter selon l'une des revendications 1 à 3,
caractérisé par le fait
que les pointes des aiguilles présentent une face extrême dont la perpendiculaire s'étend, dans la position déployée, en décrivant un angle par rapport à la direction radiale dudit cathéter.

5. Cathéter selon l'une ou plusieurs des revendications 1 à 4,
caractérisé par le fait
que les aiguilles (26), pouvant coulisser dans le sens longitudinal, sont soumises à une précharge ou à une courbure propre ;
et que, lors du coulissement des aiguilles d'injection (26) dans le sens longitudinal à travers le fût extérieur (12) du cathéter, les pointes desdites aiguilles se déplacent jusqu'à une position saillant latéralement.

6. Cathéter (10) pour injecter, respectivement, un fluide ou un médicament dans des organes creux ou des cavités corporelles, en particulier dans des vaisseaux coronariens et des artères, comprenant une pointe (14) pouvant être introduite dans des artères et un fût extérieur (12) ; une aiguille d'injection (26) logée à coulissement dans la pointe (14) du cathéter, la pointe de l'aiguille étant située à l'intérieur de la pointe du cathéter dans une position rétractée, et faisant saillie au-delà de la pointe du cathéter dans une position déployée en vue de l'administration du fluide ou du médicament ; un dispositif d'actionnement implanté à l'extrémité extracorporelle et comportant des première et seconde parties, le fût extérieur du cathéter étant fixé à l'une des parties du dispositif d'actionnement, et les deux parties étant maintenues dissociées par une force antagoniste, dispositif d'actionnement à l'aide duquel la pointe de l'aiguille peut être animée d'un coulissement à travers le fût extérieur (12) du cathéter ; et un orifice (28) prévu dans la pointe (14) du cathéter et associé à un guide-aiguille qui autorise une sortie de l'aiguille d'injection (26) dirigée tant latéralement, que vers l'avant,
caractérisé par le fait
que le fût extérieur du cathéter est réalisé sous la forme d'un flexible (120) à plusieurs canaux intérieurs, et est fixé à l'une (134) des parties du dispositif d'actionnement (132) ; que plusieurs aiguilles d'injection (26) sont prévues et sont pourvues de prolongements tubulaires (126) qui s'étendent à travers un canal intérieur respectif du flexible (120) à plusieurs canaux intérieurs, et sont fixés à l'autre partie (135) du dispositif d'actionnement (132) ;
que, lors de la compression des deux parties s'opposant à la force antagoniste, les prolongements (126), guidés dans le flexible (120) à plusieurs canaux intérieurs, déplacent les aiguilles d'injection (26) de la position rétractée à la position saillant vers l'extérieur, le fluide ou le médicament pouvant être simultanément délivré aux aiguilles d'injection (26) par l'intermédiaire de l'autre partie (135), ainsi que par l'intermédiaire des prolongements (126) ;
et qu'un fil métallique de guidage (5) est guidé tant à l'intérieur du flexible à plusieurs canaux intérieurs, qu'à travers la pointe du cathéter.

7. Cathéter selon la revendication 6,
caractérisé par le fait
que les prolongements coulissants (126) des aiguilles d'injection (26) s'étendent jusqu'à la pointe dudit cathéter.

8. Cathéter (10) pour injecter, respectivement, un fluide ou un médicament dans des organes creux ou des cavités corporelles, en particulier dans des vaisseaux coronariens et des artères, comprenant une pointe (14) pouvant être introduite dans des artères et un fût extérieur (12) ; une aiguille d'injection (26) logée à coulissement dans la pointe (14) du cathéter, la pointe de l'aiguille étant située à l'intérieur de la pointe du cathéter dans une position rétractée, et faisant saillie au-delà de la pointe du cathéter dans une position déployée en vue de l'administration du fluide ou du médicament ; un dispositif d'actionnement implanté à l'extrémité extracorporelle et comportant des première et seconde parties, le fût extérieur du cathéter étant fixé à l'une des parties du dispositif d'actionnement, et les deux parties étant maintenues dissociées par une force antagoniste, dispositif d'actionnement à l'aide duquel la pointe de l'aiguille peut être animée d'un coulissement à travers le fût extérieur (12) du cathéter ; et un orifice (28) prévu dans la pointe (14) du cathéter et associé à un guide-aiguille qui autorise une sortie de l'aiguille d'injection (26) dirigée tant latéralement, que vers l'avant,
caractérisé par le fait
que le fût extérieur du cathéter est réalisé sous la forme d'un flexible (220) à plusieurs canaux intérieurs, et est fixé à l'une (234) des parties du dispositif d'actionnement (232) ; que plusieurs aiguilles d'injection (260) sont prévues et sont pourvues de prolongements tubulaires (226) qui, s'étendant à travers un canal intérieur respectif du flexible (220) à plusieurs canaux intérieurs, sont fixés à l'autre partie (235) du dispositif d'actionnement (232) ;
que, lors de la compression des deux parties s'opposant à la force antagoniste, les prolongements (226), guidés dans le flexible (220) à plusieurs canaux intérieurs, déplacent les aiguilles d'injection (260) de la position rétractée à la position saillant vers l'extérieur, le fluide ou le médicament pouvant être simultanément délivré aux aiguilles d'injection (260) par l'intermédiaire de l'autre partie (235) et des prolongements (226) ;
qu'un ballonnet (211), placé sur la pointe (214) du cathéter destinée à l'injection, peut être gonflé et dégonflé par l'intermédiaire d'un conduit (216) du flexible (220) à plusieurs canaux intérieurs ;
et qu'un fil métallique de guidage (215) est guidé dans le ballonnet (211), par un canal intérieur (217), jusqu'à la pointe dudit ballonnet.

9. Cathéter selon la revendication 8,
caractérisé par le fait
que le fil métallique de guidage (215) est guidé, jusqu'à la pointe, soit à l'intérieur du flexible (220) à plusieurs canaux intérieurs et à travers la pointe (214) du cathéter, ainsi qu'à travers le ballonnet (211) ; soit à l'extérieur dudit flexible (220) à plusieurs canaux intérieurs, jusqu'au ballonnet (211), puis seulement dans ledit ballonnet, par le canal intérieur (217).

10. Cathéter selon la revendication 8,
caractérisé par le fait
que des fils métalliques de rigidification (230) sont intégrés, par injection, dans le matériau du flexible à plusieurs canaux intérieurs.

11. Cathéter selon l'une ou plusieurs des revendications 1 à 10,
caractérisé par le fait
que des orifices (28) munis de guide-aiguilles sont pratiqués dans la pointe (14 ; 214) dudit cathéter et guident les aiguilles d'injection, à la sortie, en les dirigeant tant latéralement que vers l'avant.

12. Cathéter (10) pour injecter, respectivement, un fluide ou un médicament dans des organes creux ou des cavités corporelles, en particulier dans des vaisseaux coronariens et des artères, comprenant une pointe (14) pouvant être introduite dans des artères et un fût extérieur (12) ; une aiguille d'injection (54) logée à coulissement dans la pointe (14) du cathéter, la pointe de l'aiguille étant située à l'intérieur de la pointe du cathéter dans une position rétractée, et faisant saillie au-delà de la pointe du cathéter dans une position déployée en vue de l'administration du fluide ou du médicament ; un dispositif d'actionnement implanté à l'extrémité extracorporelle et comportant des première et seconde parties, le fût extérieur du cathéter étant fixé à l'une des parties du dispositif d'actionnement, et les deux parties étant maintenues dissociées par une force antagoniste, dispositif d'actionnement à l'aide duquel la pointe de l'aiguille peut être animée d'un coulissement à travers la pointe du cathéter ; et un orifice (52) prévu dans la pointe du cathéter et associé à un guide-aiguille qui autorise une sortie de l'aiguille d'injection (54) dirigée tant latéralement, que vers l'avant,
caractérisé par le fait
que plusieurs aiguilles d'injection (54) sont prévues et sont installées sur un flexible interne (56), situé à l'extérieur d'un flexible intérieur (58), l'espace intercalaire pouvant être sollicité par une pression de manière à provoquer l'expansion du flexible interne (56), et à faire coulisser les aiguilles à travers la pointe du cathéter ;
et qu'un fil métallique de guidage (5) est guidé tant à l'intérieur du flexible interne (56), qu'à travers la pointe du cathéter.
